# EUROPÄISCHE PATENTSCHRIFT

(11) **EP 0 943 601 B1**
(45) Veröffentlichungstag und Bekanntmachung des Hinweises auf die Patenterteilung: **02.05.2003**
(21) Anmeldenummer: 99250052.0
(22) Anmeldetag: 25.02.1999
(51) Int. Cl.: C07C 69/743, A61K 6/083, C08F 36/20

(54) **Vinylcyclopropan-(Meth) acrylate und deren Verwendung**
Vinylcyclopropane-(meth)acrylates as well as their use
(Méth)-acrylates du vinylcyclopropane ainsi que leur utilisation

(30) Priorität: 17.03.1998 DE 19812888
(43) Veröffentlichungstag der Anmeldung: 22.09.1999
(73) Patentinhaber: Ivoclar Vivadent AG, 9494 Schaan (LI)
(72) Erfinder: Moszner, Norbert, Prof.Dr., 9492 Eschen (LI); Rheinberger, Volker, Dr., 9490 Vaduz (LI); Völkel, Thomas, Dr., 88131 Lindau (DE); Fischer, Urs Karl, 9320 Arbon (CH)
(74) Vertreter: UEXKÜLL & STOLBERG

(56) Entgegenhaltungen:
- EP-A- 0 798 286
- EP-A- 0 867 444
- DE-A- 19 714 320

## Beschreibung

Die Erfindung betrifft Vinylcyclopropan-Derivate mit polymerisierbaren (Meth)acrylat-Resten, Verfahren zu deren Herstellung und Dentalmaterialien auf der Basis dieser Verbindungen.

1,1-Disubstituierte 2-Vinylcyclopropane haben aufgrund ihres geringen Schrumpfes bei der radikalischen Polymerisation Interesse gefunden. F. Sanda, T. Takata, T. Endo, Macromolecules 26 (1993) 1818), haben z.B. gezeigt, daß die flüssigen Monomere 1,1-Bis(ethoxycarbonyl)- oder 1,1-Dicyano-2-vinylcylopropan im Vergleich zu herkömmlichen Vinylmonomeren, wie z.B. Acrylnitril oder Methylmethacrylat, bei der Polymerisation einen geringen Volumenschrumpf ergeben.

J. Sugiyama, K. Ohashi, M. Ueda, Macromolecules 27 (1994) 5543 beobachteten bei der radikalischen Polymerisation von 1,1-Bis(phenoxycarbonyl)-2-vinylcyclopropan sogar eine Volumenexpansion. Nachteilig an den bekannten Vinylcyclopropanen ist jedoch ihr geringes Polymerisationsvermögen. Bei der Copolymerisation von 1,1-Bis(ethoxycarbonyl) -2-vinylcyclopropan mit Methylmethacrylat (MMA) werden beispielsweise nur heterogen zusammengesetzte Produkte mit einer geringen Einbindung des Vinylcylopropan-Derivates in die Copolymerstruktur erhalten (F. Sanda, T. Takata, T. Endo, Macromolecules 27 (1994) 3982), so daß die mechanischen Eigenschaften der Polymere nur unbefriedigend sind.

Darüber hinaus sind Vinylcyclopropane mit mehreren polymerisationsfähigen Gruppen bekannt. F. Sanda, T. Takata, T. Endo, Macromolecules 27 (1994) 3986, beschreiben 1-Vinyl-5,7-dioxaspiro[2.5]octan-6-on, ein Hybridmonomer, das eine Vinylcyclopropanund eine cyclische Carbonat-Gruppe enthält, und T. Okazaki, F. Sanda, T. Endo, Macromolecules 28 (1995) 6026, 1,10-Bis(vinyl)-4,8,12,15-tetraoxatrispiro[2.2.2.2.2.2]pentadecan, ein Monomer, bei dem zwei Vinylcyclopropangruppen über eine Spiroacetal-Einheit miteinander verbunden sind. Diese Verbindungen sind hydrolyseempfindlich und weisen im Vergleich zu monofunktionellen Vinylcyclopropanen keine verbesserte radikalische Copolymerisationsfähigkeit mit (Meth)acrylverbindungen auf.

Die EP 0 798 286 Al offenbart multifunktionelle, radikalisch polymerisierbare Vinylcyclopropan-Derivate, die mindestens zwei Vinylcyclopropan-Einheiten aufweisen und die sich insbesondere zur Herstellung von Dentalmaterialien eignen.

Hybridmonomere, die neben einer Vinylcyclopropan-Gruppe auch eine (Meth)acrylatgruppe enthalten, sind nicht bekannt.

Der Erfindung liegt die Aufgabe zugrunde, polymerisierbare Vinylcyclopropane bereitzustellen, die bei der Polymerisation einen geringen Volumenschrumpf zeigen und gleichzeitig radikalisch gut mit Acrylaten oder Methacrylaten copolymerisierbar sind.

Diese Aufgabe wird überraschend durch hybride Vinylcyclopropan-Methacrylate gemäß der allgemeinen Formel (I) gelöst, in der U, X, Y, Z, R¹, R², R³, R⁴, n und m unabhängig voneinander die folgenden Bedeutungen haben: 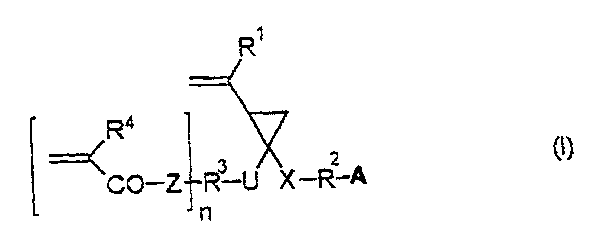
- A: -[-Y-CO-C(=CH₂)R⁴]ₘ oder -C(=CH₂)CO-O-C₁₋₄-Alkyl, wobei Alkyl vorzugsweise Methyl oder Ethyl ist;
- U, X: CO, COO oder CONH;
- Y, Z: O, NH oder entfallen, wobei jedoch Y und Z nicht gleichzeitig entfallen können wenn A gleich -[-Y-CO-C(=CH₂)R⁴]ₘ ist;
- n, m: eine ganze Zahl von 0 bis 4, wobei n und m nicht gleichzeitig 0 sind;
- R¹: H, CH₃ oder Cl;
- R², R³: H, C₁- bis C₁₀-Alkyl bzw. Alkylen, das durch O, S oder NH unterbrochen sein kann, C₆- bis C₁₄-Aryl bzw. Arylen, C₇- bis C₁₄-Alkylaryl bzw. Alkylarylen oder C₅- bis C₈-Cycloalkyl bzw. Cycloalkylen; und
- R⁴: H oder ein C₁- bis C₁₀-Alkyl.

Unabhängig voneinander wählbare bevorzugte Definitionen sind:
- A: -[-Y-CO-C(=CH₂)R⁴]ₘ
- U, X: COO oder CONH, insbesondere COO,
- Y, Z: O oder entfällt,
- n, m: eine ganze Zahl von 0 bis 2, wobei Derivate bei denen n = 1 oder 2 und m = 0 ist, besonders bevorzugt sind,
- R¹: H,
- R², R³: C₁- bis C₆-Alkyl bzw. Alkylen, C₆-Aryl bzw. Arylen oder C₆-Cycloalkyl bzw. Cycloalkylen, insbesondere C₁- bis C₃-Alkyl bzw Alkylen, Phenyl oder Phenylen, insbesondere p-Phenylen, Cyclohexyl oder Cyclohexylen, insbesondere p-Cyclohexylen und/oder
- R⁴: H, C₁-C₃-Alkyl, insbesondere CH₃.

Besonders bevorzugt sind Vinylcyclopropan-Derivate bei denen
- R² und R³: gleich sind,
- R⁴: H ist,
- n,m: jeweils 1 sind und
- U, X: COO oder CONH bedeuten,
oder
- R² und R³: verschieden sind,
- R⁴: H ist,
- n: 0 ist,
- m: 1 oder 2 ist,
- Z: entfällt und
- Y: O bedeutet.

Die übrigen Substituenten und Indices haben die oben angegebenen Bedeutungen.

Ganz besonders bevorzugte Vinylcyclopropan-(Meth)acrylate der Formel (I) sind:

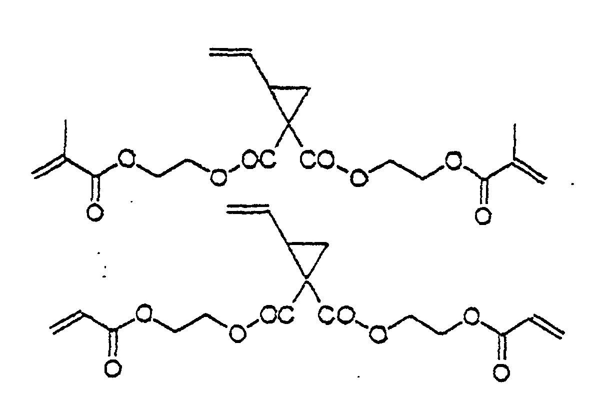

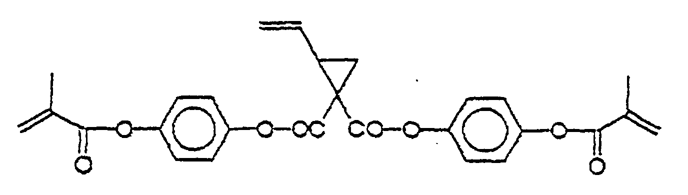

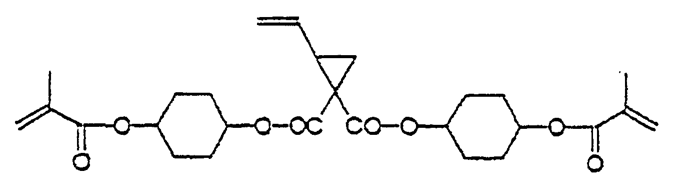

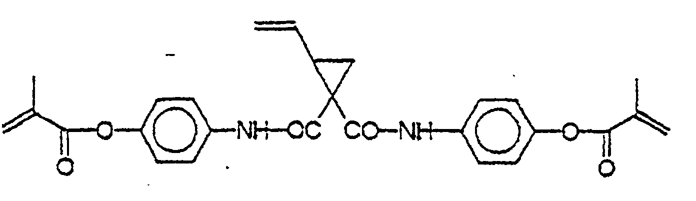

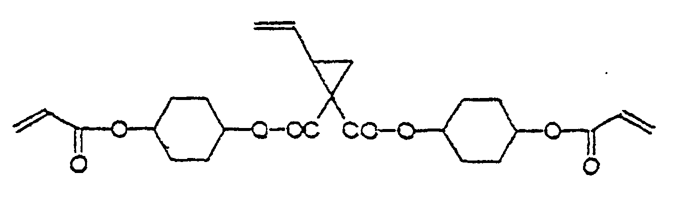

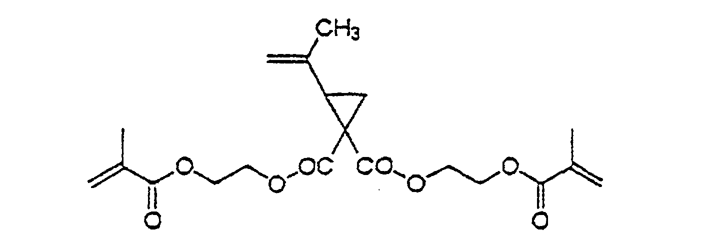

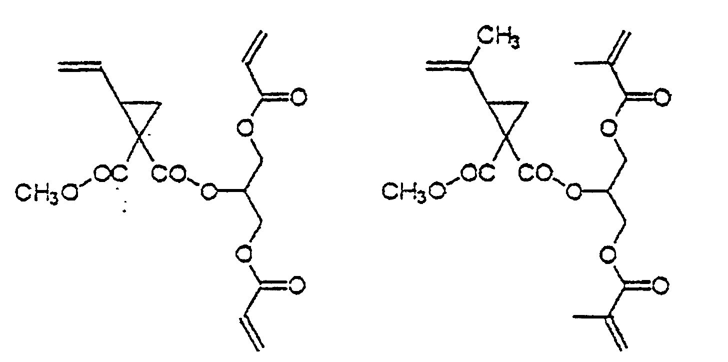

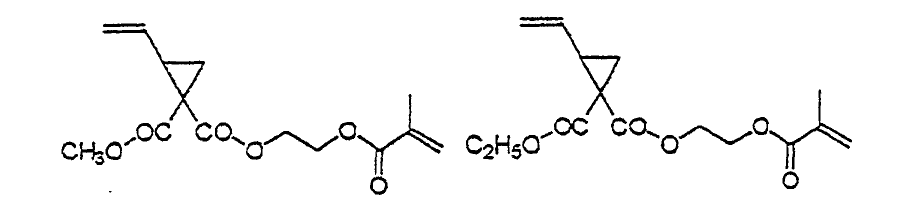

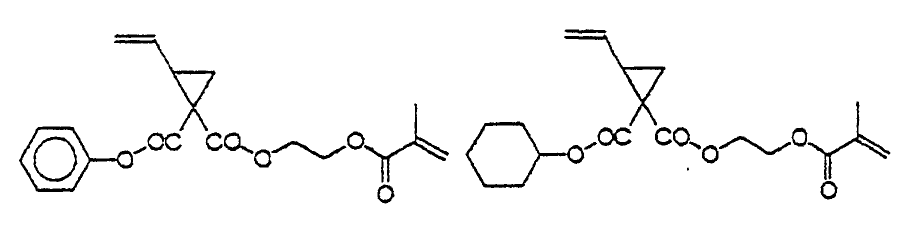

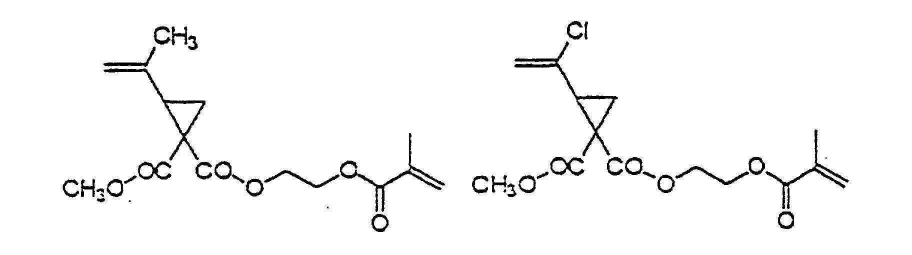

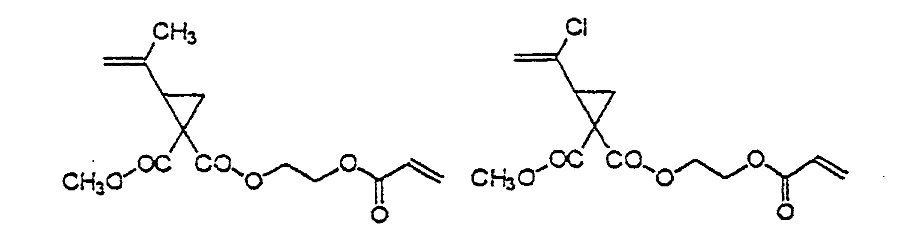

Die erfindungsgemäßen Vinylcyclopropan-(Meth)acrylate (I) lassen sich herstellen, indem Vinylcyclopropan-Derivate der Formel (II) mit (Meth)acrylsäurederivaten (III), bei denen W die Bedeutung OH oder Halogen, vorzugsweise Br oder Cl hat, schrittweise zu den Zwischenprodukten (IV) umgesetzt werden, die dann mit weiterem (Meth)arcylsäurederivat (III) in die gewünschten Vinylcyclopropan-Derivate überführt werden. Ist das im zweiten Reaktionsschritt verwendete (Meth)acrylsäurederivat (III) mit dem im ersten Schritt verwendeten Derivat identisch, werden symmetrische Vinylcyclopropan-(Meth)acrylate erhalten, durch die Verwendung eines strukturverschiedenen Derivats (III) sind unsymmetrische Verbindungen (I) zugänglich.

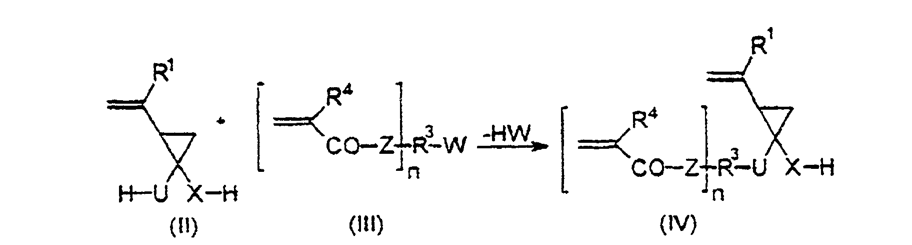

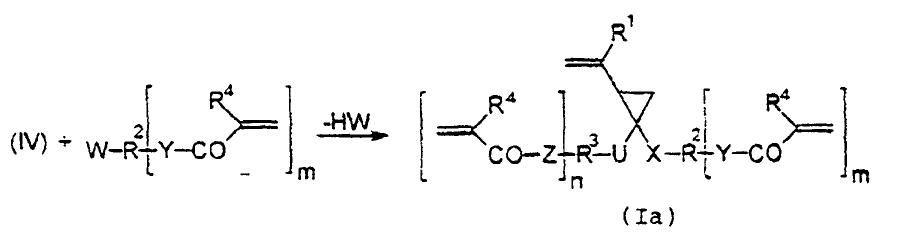

Beispielsweise kann das Vinylcyclopropan-Methacrylat (1) durch Veresterung von 2 mol 2-Hydroxyethylmethacrylat (HEMA) mit 1 mol 2-Vinylcyclopropan-1,1-dicarbonsäure, die durch Verseifung von 1,1-Dimethoxycarbonyl-2-vinylcyclopropan zugänglich ist, in Gegenwart von Dicyclohexylcarbodiimid (DCC) erhalten werden.

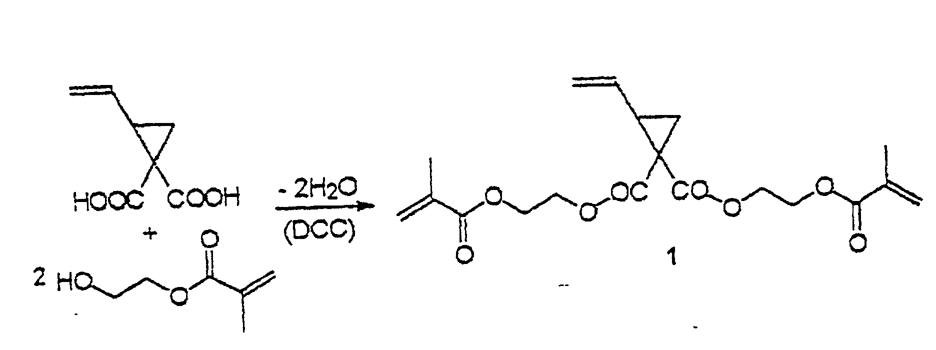

Vinylcyclopropan-(Meth)acrylate bei denen n oder m die Bedeutung 0 haben lassen sich herstellen, indem unsymmetrisch substituierte 2-Vinylcyclopropanderivate (VI), in denen R⁵ vorzugsweise ein C₁- bis D₁₀-Alkyl- oder Phenylrest ist, analog mit 1 mol (Meth)acrylsäurederivat (III) zu den Vinylcyclopropan-(Meth)acrylaten (V) umgesetzt werden.

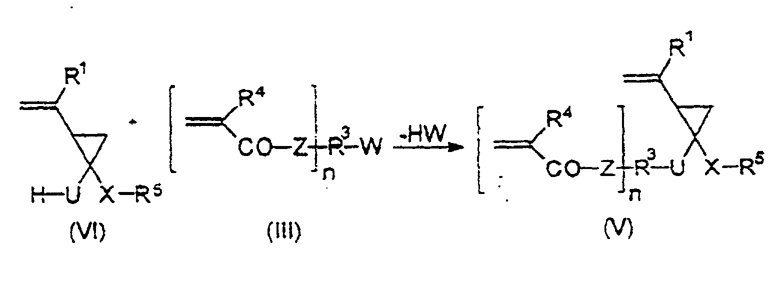

Beispielsweise kann das Vinylcyclopropan-Methacrylat (2) durch Veresterung von Glycerindimethacrylat mit 1 mol 2-Vinylcyclopropan-1-methoxycarbonyl-1-carbonsäure, die durch partielle Verseifung von 1,1-Dimethoxycarbonyl-2-vinylcyclopropan zugänglich ist, in Gegenwart von Dicyclohexylcarbodiimid (DDC) erhalten werden.

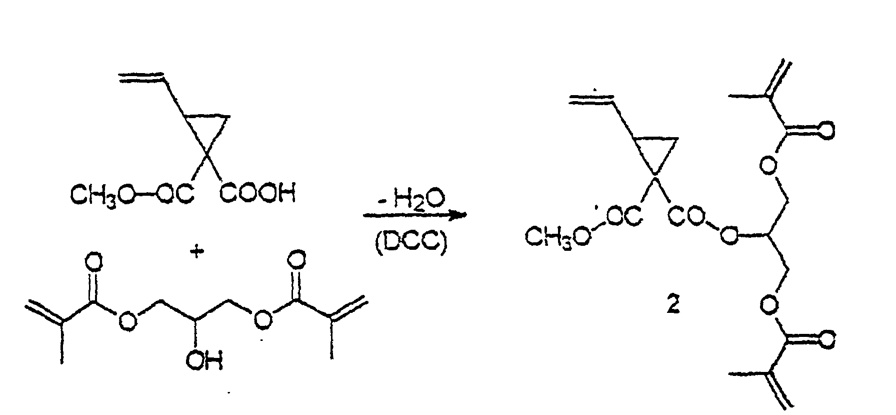

Die Verbindungen der Formel (II) können analog den für die Herstellung von Vinylcyclopropanen bekannten Verfahren (vgl. z.B. US-A-4,713,478 und US-A-4,713,479) durch Umsetzung von unsubstituierten (R¹ = entfällt) oder in 2-Stellung substituierten trans-1,4-Dibrombut-2-enen mit entsprechenden Malonsäurederivaten hergestellt werden, wobei gegebenenfalls auf die Schutzgruppen-Technik zurückgegriffen werden muß.

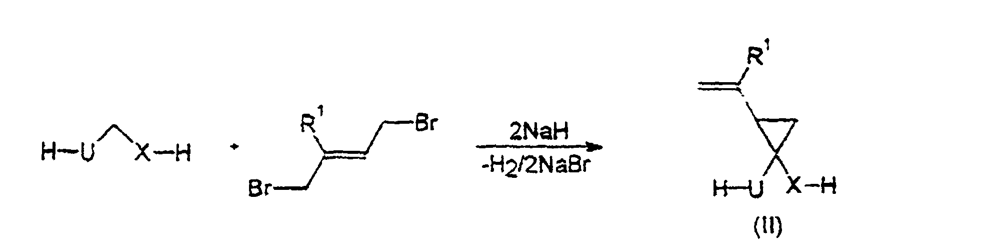

Als (Meth)acryl-Derivate (III) können bekannte OH- oder NH₂gruppenhaltige (Meth)acrylverbindungen eingesetzt werden. Bevorzugte Verbindungen sind 2-Hydroxyethyl(meth)acrylat, 2-Hydroxypropyl(meth)acrylat, Glycerindi(meth)acrylat, oligomere Ethylenglycolmono(meth)acrylate, 3-Hydroxyphenyl(meth)acrylat, 4-Hydroxybenzyl(meth)acrylat, 4-Hydroxycyclohexyl(meth)acrylat, 2-Aminoethyl(meth)acrylat, 4-Aminophenyl(meth)acrylat oder Triethylendiaminmono(meth)acrylat.

Solche (Meth)acryl-Edukte lassen sich einfach z.B. durch Umsetzung von bekannten zwei- oder mehrwertigen Alkoholen, di- oder multifunktionellen Aminoverbindungen oder Aminoalkoholen, die mindestens eine Anzahl von p OH- oder NH₂-Gruppen im Molekül enthalten, wobei p ≥ 2 ist, mit (p-1) mol (Meth)acrylsäurechlorid oder -anhydrid herstellen oder sind durch Umsetzung von r mol Glycidyl(meth)acrylat mit Verbindungen, die pro Molekül r OH-, NH₂- bzw. COOH-Gruppen enthalten, zugänglich, wobei r ≥ 1 ist.

Die erfindungsgemäßen Vinylcyclopropan-Derivate eignen sich besonders zur Herstellung von Adhäsiven, Zementen, Kompositen oder Formkörpern, wie z.B. Linsen oder Scheiben, insbesondere zur Herstellung von Dentalmaterialien.

Hierzu werden die erfindungsgemäßen Monomere mit einem Initiator für die radikalische Polymerisation und vorzugsweise auch mit zusätzlichen Monomeren, Füllstoffen und ggf. weiteren Hilfsstoffen gemischt.

Bei der Polymerisation ergeben die erfindungsgemäßen Verbindungen aufgrund ihrer hybriden Monomerstruktur entweder lineare Poly[(meth)acrylate] mit seitenständigen 2-Vinylcyclopropan-Gruppen oder vernetzte Polymere.

Als Initiatoren für die radikalische Polymerisation eignen sich die bekannten Initiatoren für die Kalt-, Heiß- und Photohärtung. Geeignete Initiatoren werden beispielsweise in der Encyclopedia of Polymer Science and Engineering, Vol. 13, Wiley-Intersci. Pub., New York etc. 1988, S. 754 ff. beschrieben.

Bevorzugte Initiatoren sind Azoverbindungen, wie Azobis(isobutyronitril) (AIBN) oder Azobis(4-cyanvaleriansäure) oder Peroxide, wie Dibenzoylperoxid, Dilauroylperoxid, tert.-Butylperoctoat, tert.-Butylperbenzoat oder Di-(tert.-butyl)-peroxid.

Als Initiatoren für die Heißhärtung eignen sich besonders Benzpinakol und 2,2'-Di(C₁-C₈-alkyl)benzpinakole.

Geeignete Photoinitiatoren für den UV- oder sichtbaren Bereich werden von J.P. Fouassier, J.F. Rabek (Hrsg.), Radiation Curing in Polymer Science and Technology, Vol. II, Elsevier Applied Science, London und New York 1993, Seiten 155 bis 237, beschrieben. Bevorzugte Photoinitiatoren sind Benzoinether, Dialkylbenzilketale, Dialkoxyacetophenone, Acylphosphinoxide, α-Diketone, wie 10-Phenanthrenchinon, Diacetyl, Furil, Anisil, 4,4'-Dichlorbenzil und 4,4'-Dialkoxybenzil und Kampferchinon.

Zur Herstellung von Dentalmaterialien sind Dibenzoylperoxid, Kampferchinon und Acylphosphinoxide bevorzugt.

Bevorzugte zusätzliche Monomere für die Herstellung von Klebstoffen oder Dentalmaterialien sind vernetzende bi- oder mehrfunktionelle Acrylate bzw. Methacrylate, wie z.B. Bisphenol-Adi(meth)acrylat, Bis-GMA, ein Additionsprodukt aus Methacrylsäure und Bisphenol-A-diglycidylether, 7,7,9-Trimethyl-4,13-dioxo-3,14-dioxa-5,12-diazahexadecan-1,16-diyl-dimethacrylat (UDMA), ein Additionsprodukt aus Hydroxyethylmethacrylat und 2,2,4-Trimethylhexamethylendiisocyanat, Di-, Tri- oder Tetraethylenglycoldi(meth)acrylat, Decandioldi(meth)acrylat, Trimethylolpropantri(meth)acrylat, Pentaerythrittetra(meth)acrylat, Butandioldi(meth)acrylat, 1,10-Decandioldi(meth)acrylat, 1,12-Dodecandioldi(meth)acrylat, die durch Veresterung von (Meth)acrylsäure mit den entsprechenden Diolen zugänglich sind, sowie di- und mehrfunktionelle 2-Vinylcyclopropanderivate, die durch Umsetzung von 1-Methoxycarbonyl-2-vinylcyclopropan-1-carbonsäure mit zweioder mehrwertigen OH- oder NH₂-Verbindungen als Kopplungskomponente, d.h. z.B. mit Ethylenglycol, Di- oder Triethylenglycol, Butylenglycol, 1,6-Hexandiol, Glycerin, Triethanolamin, Trimethylolpropantriol, Pentaerythrit oder Glucose, sowie Hydrochinon, Resorcin, Brenzkatechin oder Pyrogallol, Ethylendiamin, Propylendiamin, Hexamethylendiamin, o-, p- oder m-Phenylendiamin, zugänglich sind.

Als Füllstoffe zur Verbesserung der mechanischen Eigenschaften eignen sich alle organischen und anorganischen Partikel oder Fasern.

Bevorzugte Füllstoffe zur Herstellung von Dentalmaterialien wie Befestigungszemente oder Füllungskomposite sind amorphe, kugelförmige Materialien auf der Basis von Mischoxiden aus SiO₂, ZrO₂ und/oder TiO₂ mit einer mittleren durchschnittlichen Partikelgröße von 0,005 bis 2,0 µm, vorzugsweise von 0,1 bis 1 µm, wie sie beispielsweise in der DE-PS 32 47 800 offenbart werden, mikrofeine Füllstoffe, wie pyrogene Kieselsäure oder Fällungskieselsäure sowie Makro- oder Mini-Füllstoffe, wie Quarz-, Glaskeramik- oder Glaspulver mit einer durchschnittlichen Teilchengröße von 0,01 bis 20 µm, vorzugsweise 0,5 bis 5 µm, sowie röntgenopake Füllstoffe, wie Ytterbiumfluorid. Unter Mini-Füllstoffen werden Füllstoffe mit einer Partikelgröße von 0,5 bis 1,5 µm und unter Makro-Füllstoffen Füllstoffe mit einer Partikelgröße von 10 bis 20 µm verstanden. Außerdem können auch Glasfasern, Polyamid- oder Kohlenstoff-Fasern eingesetzt werden.

Darüber hinaus können die erfindungsgemäßen Zusammensetzungen im Bedarfsfall weitere Hilfsstoffe enthalten, insbesondere Stabilisatoren, UV-Absorber, Farbstoffe, Pigmente und/oder Gleitmittel. Unter Stabilisatoren werden solche Stoffe verstanden, die eine vorzeitige Polymerisation verhindern und damit vor allem die Lagerstabilität von Monomermischungen und Kompositen erhöhen, ohne jedoch die Eigenschaften der ausgehärteten Materialien zu beeinträchtigen. Bevorzugte Stabilisatoren sind Hydrochinonmonomethylether (MEHQ) und 2,6-Di-tert.-butyl-4-methylphenol (BHT).

Werden die erfindungsgemäßen Vinylcyclopropan-Methacrylate mit monofunktionellen (Meth)acrylaten in Lösung anionisch oder radikalisch copolymerisiert, so lassen sich lösliche Copolymere erhalten, die seitenständig gebundene 2-Vinylcyclopropangruppen tragen und die in einer zweiten Stufe z.B. als Substanzfilm zu entsprechenden unlöslichen Netzwerkpolymerfilmen umgewandelt werden können. Die löslichen Copolymere und Filme können beispielsweise als strahlenvernetzbare Lacke oder Klebstoffe verwendet werden.

Die erfindungsgemäßen Vinylcyclopropan-(Meth)acrylate eignen sich besonders zur Herstellung von Dentalmaterialien, wie Dentaladhäsive, Befestigungszemente oder Füllungskomposite, sowie Materialien für Inlays/Onlays, Zähne oder Verblendmaterialien für Kronen und Brücken.

Bevorzugte Dentalmaterialien enthalten:
(a) 1 bis 99 Gew.-%, bevorzugt 5 bis 70 Gew.-% und besonders bevorzugt 5 bis 40 Gew.-% der erfindungsgemäßen Vinylcyclopropan-(Meth)acrylate,
(b) 0,01 bis 5 Gew.-%, besonders bevorzugt 0,1 bis 2,0 Gew.-% an radikalischem Initiator,
(c) 0 bis 80 Gew.-%, bevorzugt 0 bis 60 Gew.-% und besonders bevorzugt 0 bis 50 Gew.-% an anderen radikalisch polymerisierbaren Monomerkomponenten,
(d) 0 bis 90 Gew.-% Füllstoff.

Der bevorzugte Füllstoffgehalt richtet sich nach dem Anwendungszweck und beträgt im Fall von Adhäsiven 0 bis 20 Gew.-%, im Fall von Zementen 20 bis 60 Gew.-% und im Fall von Füllungskompositen 60 bis 85 Gew.-%.

Die erfindungsgemäßen Dentalmaterialien zeichnen sich durch einen geringeren Polymerisationsschrumpf und gute mechanische Eigenschaften aus.

Im folgenden wird die Erfindung anhand von Ausführungsbeispielen näher erläutert.

### Beispiele

### Beispiel 1

### 2-Vinylcyclopropan-1,1-dicarbonsäuremonomethylester (3)

In einem 100 ml Zweihalskolben mit Thermometer, Magnetrührer und CaCl₂-Rohr wurden 36,8 g (0,2 mol) 2-Vinylcylopropan-1,1-dicarbonsäuredimethylester, der aus Malonsäuredimethylester und trans-1,4-Dibrombut-2-en zugänglich ist (vgl. US-A-4,713,478 und US-A-4,713,479), in 65 ml Methanol gelöst und mit Eis-Wasser auf ca. 5°C gekühlt. Die Mischung wurde dann portionsweise mit 13,3 g (0,2 mol) KOH versetzt, so daß die Temperatur nicht über 15°C anstieg. Zur Vervollständigung der Reaktion wurde für weitere 12 h gerührt und die Mischung dann am Rotationsverdampfer im Vakuum (50 mbar) bei 50°C eingeengt. Das erhaltene Öl (ca. 43 g) wurde in 50 ml Wasser gelöst und unter Kühlung mit konz. Salzsäure auf etwa pH 2-3 eingestellt. Die organische Phase wurde in 100 ml Diethylether aufgenommen, die wäßrige Phase noch zweimal mit je 100 ml Diethylether extrahiert und die vereinigten Etherphasen über wasserfreiem Na₂SO₄ getrocknet. Die Lösung wurde mit 0,01 g Hydrochinonmonomethylether stabilisiert, im Vakuum eingeengt und im Feinvakuum getrocknet. Es wurden 28,2 g (83% Ausbeute) einer farblosen Flüssigkeit erhalten.

| | | | | |
|---|---|---|---|---|
| Elementaranalyse | C₈H₁₀O₄ | Ber. | C 56,47 | H 5,92 |
| | (170,2) | Gef. | C 56,60 | H 5,82 |

IR (Film, cm⁻¹): 921 (m, sh), 1145 (s), 1209 (s, sh), 1289 (m), 1333 (s, sh), 1440 (s, sh), 1777 (s, sh), 2957 (M) und 3018 (m).
¹H-NMR (90 MHz, CDCl₃): 1,93 und 2,03 (s, 2 x 1H, CH₂-Cyclopr.); 2,88-2,90 (q, 1H, CH-Cyclopr.); 3,86 (s, 3H, CH₃); 5,18-5,69 (m, 3H, CH=CH₂); 12,30 (s, 1H, COOH, H/D-Aust.).
¹³C-NMR (75 MHz, CDCI₃, ppm): 171,9 und 171,7 (=O), 132,4 (CH=CH₂), 120,1 (CH=CH₂), 53,1 (OCH₃), 37,1 (C-CO), 34,2 (CH-Cyclopr.) und 22,8 (CH₂-Cyclopr.).

### Beispiel 2

### 2-(1-Carboxymethyl-2-vinylcyclopropan-1-carboxy)-ethylmethacrylat (4)

Zu einer Lösung von 53 g (0,31 mol) 2-Vinylcyclopropan-1,1-dicarbonsäuremonomethylester, 40 g (0,31 mol) 2-Hydroxyethylmethacrylat, 1,0 g 4-Dimethylaminopyridin in 160 ml Methylenchlorid wurden bei 0 bis 10°C portionsweise 64 g (0,31 mol) Dicyclohexylcarbodiimid unter Rühren zugegeben. Nach 3 h Rühren bei 5°C wurde der gebildete Niederschlag an Dicyclohexylharnstoff abfiltriert und das Filtrat mit 0,5 N Salzsäure und anschließend mit Wasser gewaschen. Nach dem Trocknen über wasserfreiem Na₂SO₄ wurde das Lösungsmittel im Wasserstrahlvakuum abgezogen und das zurückbleibende Produkt im Feinvakuum fraktioniert destilliert. Es wurden 38 g (43% Ausbeute) einer farblosen Flüssigkeit erhalten (Kp._{0,2}:145°C).

| | | | | |
|---|---|---|---|---|
| Elementaranalyse | C₁₄H₁₈O₆ | Ber. | C 59,55 | H 6,43 |
| | (282,4) | Gef. | C 59,90 | H 6,59 |

IR (Film, cm⁻¹): 918 (w), 1134 (m), 1168 (s), 1272 (m), 1438 (m), 1638 (w), 1726 (s) und 2956 (w).
¹H-NMR (400 MHz, CDCI₃, ppm): 1,58-1,78 (m, 2H, CH₂-Cyclopr.); 1,95 (s, 3H, CH₃); 2,56-2,63 (m, 1H, CH-Cyclopr.); 3,73 (s, 3H, OCH₃); 4,33-4,47 (m, 4H, CH₂CH₂); 5,11-5,48 (m, 3H, CH=CH₂); 5,60 und 6,13 (2s, 2H, =CH₂).
¹³C-NMR (75 MHZ, CDCI₃, ppm): 169,1 (a), 166,8 und 167,0 (b), 136,0 (c), 133,0 (d), 126,0 (e), 118,7 (f), 63,2 und 62,1 (g,h), 52,4 (i), 35,8 (k), 31,4 (l), 20,6 (m) und 18,3 (n).

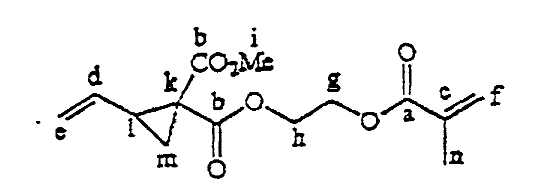

### Beispiel 3

### Synthese von 2-(1-Carboxymethyl-2-vinylcyclopropan-1-carboxy)-ethylacrylat (5)

Zu einer Lösung von 17 g (0,10 mol) 2-Vinylcyclopropan-1,1-dicarbonsäuremonomethylester, 11,6 g (0,10 mol) 2-Hydroxyethylacrylat, 0,3 g 4-Dimethylaminopyridin in 50 ml Methylenchlorid wurden bei 0 bis 10°C portionsweise 20 g (0,10 mol) Dicyclohexylcarbodiimid unter Rühren gegeben. Nach 3 h Rühren bei 5°C wurde der gebildete Niederschlag an Dicyclohexylharnstoff abfiltriert und das Filtrat mit 0,5 N Salzsäure und anschließend mit Wasser gewaschen. Nach dem Trocknen über wasserfreiem Na₂SO₄ wurde das Produkt chromatographisch gereinigt (Säule: 15 cm lang, Durchmesser 2 cm, Träger: Silica 60, Elutionsmittel: Ethylacetat/Hexan; 7:3), das Lösungsmittel anschließend im Wasserstrahlvakuum abgezogen und das zurückbleibende Produkt im Feinvakuum getrocknet. Es wurden 19 g (70% Ausbeute) einer farblosen Flüssigkeit erhalten.

| | | | | |
|---|---|---|---|---|
| Elementaranalyse | C₁₃H₁₆O₆ | Ber. | C 58,19 | H 6,01 |
| | (268,3) | Gef. | C 57,84 | H 6,12 |

IR (Film, cm⁻¹): 922 (m), 988 (m), 1072 (m), 1133 (m), 1189 (s), 1271 (s), 1408 (m), 1440 (m), 1638 (m), 1730 (s) und 2955 (m).
¹H-NMR (400 MHz, CDCI₃, ppm): 1,58-1,76 (m, 2H, CH₂-Cyclopr.); 2,57-2,63 (m, 1H, CH-Cyclopr.); 3,73 (s, 3H, OCH₃); 4,36-4,41 (m, 4H, CH₂CH₂); 5,14-5,41 (m, 3H, CH=CH₂) und 5,86-6,783 (m, 3H, CO-CH=CH₂).
¹³C-NMR (75 MHz, CDCI₃, ppm): 169,1 (a), 167,5 und 165,8 (b,c), 132,9 (d), 131,4 (e), 128,0 (f), 118,8 (g), 63,6 und 62,1 (h,i), 52,6 (j), 35,7 (l), 31,9 (m) und 20,6 (n).

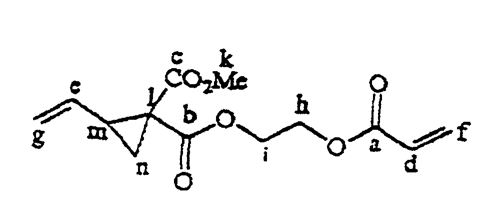

### Beispiel 4

### Synthese von 2-(1-Carboxymethyl-2-vinylcyclopropan-1-carboxy)methylacrylsäureethylester (6)

Zu einer Lösung von 13 g (70 mmol) 2-Vinylcyclopropan-1,1-dicarbonsäuremonomethylester, 9,2 g (70 mmol) 2-Hydroxymethylacrylsäureethylester und 0,1 g 4-Dimethylaminopyridin in 40 ml Methylenchlorid wurden bei 0 bis 10 °C 14,5 g (70 mmol) Dicyclohexylcarbodiimid portionsweise unter Rühren gegeben. Nach 8 h Rühren bei Raumtemperatur wurde der gebildete Niederschlag an Dicyclohexylharnstoff abfiltriert, das Filtrat mit 0,5 N Salzsäure und anschließend mit Wasser gewaschen und über wasserfreiem Na₂SO₄ getrocknet. Anschließend wurde das Produkt chromatographisch gereinigt (Säule: 15 cm lang, Durchmesser 2 cm, Träger: Silica 60, Elutionsmittel: Ethylacetat/Hexan: 7,3), das Lösungsmittel im Wasserstrahlvakuum abgezogen und das zurückbleibende Produkt im Feinvakuum getrocknet. Es ergaben sich 12,4 g (62 % Ausbeute) einer farblosen Flüssigkeit.

| | | | | |
|---|---|---|---|---|
| Elementaranalyse | C₁₄H₁₈O₆ | Ber. | C 59,77 | H 6,45 |
| | (281,3) | Gef. | C 59,13, | H 6,27 |

IF(Film, cm⁻¹): 2954 (m), 1729 (s), 1640 (m), 1438 (m), 1308 (m), 1228 (s) und 1029 (m).
¹H-NMR (400 MHz, CDCl₃, ppm): 5,84 und 6,36 (2 s, 2H, =CH₂ (Acrylat)), 4,80 - 5,29 (m, 3H, CH=CH₂), 4.80 und 4,96 (2 s, 2H, OCH₂), 3,74 (s, 3H, OCH₃), 2,60 (m, 1H, CH (Cycloprop.)) und 1,60-1,75 (m, 2H, CH₂ (Cycloprop.)).
¹³C-NMR (75 MHz, CDCl₃, ppm): 14,1 (a), 23,1 (b), 31,5 (c), 35,7 (d), 52,6 (e), 60,7 (f), 63,4 (g), 119,6 (h), 127,0 (i), 132,6 (k), 135,2 (l) und 166,1-168,9 (m).

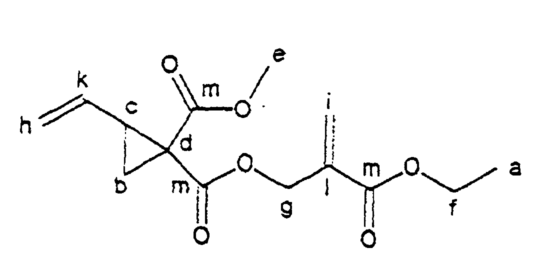

### Beispiel 5

### Radikalische Homo- und Copolymerisation von 2-(1-Carboxymethyl-2-vinylcylopropan-1-carboxy)-ethylmethacrylat

**Homopolymerisation:** In einem Schlenkgefäß wurden zu einer Lösung von 2-(1-Carboxymethyl-2-vinylcyclopropan-1-carboxy)-ethylmethacrylat (0,5 mol/l) in Chlorbenzol 2,5 mol-% (bezogen auf das Monomer) Azobisisobutyronitril (AIBN) gegeben. Nach dem Entgasen der Monomerlösung und Verschließen des Schlenkgefäßes unter Argon wurde die Mischung in einem thermostatisierten Wasserbad bei 65 °C polymerisiert. Nach 4 h wurde die Polymerisation abgebrochen, indem das Polymerisat durch Eingießen der Reaktionsmischung in die 10fache Menge Methanol ausgefällt wurde. Das gebildete Polymer wurde abfiltriert und bis zur Gewichtskonstanz getrocknet. Es wurde ein Homopolymer mit einer mittleren Molmasse von 70 000 g/mol (Zahlenmittel) in 53 % Ausbeute erhalten. Das ¹H-NMR-Spektrum zeigt, daß es sich bei dem Homopolymerisat um ein Polymethacrylat mit seitenständig gebundenen 1-Carboxymethyl-2-vinylcyclopropan-Gruppen handelt.

Analog wurde2-(1-Carboxymethyl-2-vinylcyclopropan-1-carboxy)ethylmethacrylat in Substanz mit AIBN (2,5 mol-%) bei 65 °C polymerisiert. Nach 15 h wurde ein transparentes, hartes und unlösliches Polymerisat erhalten, bei dem offensichtlich beide polymerisationsfähige Gruppen des Ausgangsmonomeren in die Polymernetzwerkbildung einbezogen waren.

**Copolymerisation:** Analog zur Homopolymerisation wurde eine Monomermischung aus 2-(1-Carboxymethyl-2-vinylcyclopropan-1-carboxy)ethylmethacrylat (0,5 mol/l), Methylmethacrylat (0,05 mol/l) und AIBN (2,5 mol-%) in Chlorbenzol hergestellt und 2 h polymerisiert. Die Ausbeute an Copolymer betrug 45 %. Das ¹H-NMR-Spektrum zeigt, daß das Copolymerisat seitenständig gebundene 1-Carboxymethyl-2-vinylcyclopropan-Gruppen enthält.

### Beispiel 6

### Herstellung eines Füllungskomposits auf der Basis von 2-(1-Carboxymethyl-2-vinylcyclopropan-1-carboxy)-ethylmethacrylat

Es wurden die folgenden Kompositfüllungsmaterialien hergestellt indem die aufgeführten Komponenten in den in Tabelle 1 angegebenen Mengen in einem Planetenkneter (Typ LPM, Zwischenraum 2SP, Fa. Linde) angemischt wurden.

**Material A (Vergleichsbeispiel):** Komposit auf Basis einer Mischung aus zwei Methacrylatvernetzern [Urethandimethacrylat RM-3 (7,7,9-Trimethyl-4,13-dioxo-3,14-dioxa-5,12-diazahexadecan-1,16-diyl-dimethacrylat; Ivoclar) und Bis-GMA (Bisphenol-A-glycidylmethacrylat; Esschem)] und einem vernetzenden Vinylcyclopropan [BVCPRE (Bis(2-vinyl-cyclopropandicarbonsäuremethyl)resorcinylester; Ivoclar)].

**Material B:** In Komposit B wurde ein Teil des Vinylcyclopropan BVCPRE durch das erfindungsgemäße Vinylcyclopropanmethacrylat (4) ersetzt.

Zur Untersuchung der mechanischen Eigenschaften wurden Prüfkörper entsprechend der ISO-Norm 4049 (1988) geformt und durch Bestrahlen mit einer dentalen Lichtquelle (Spectramat der Firma Vivadent; Wellenlänge 400 bis 500 nm; 2 × 3 Minuten) gehärtet. Anschließend wurden die mechanischen Eigenschaften gemäß ISO-Norm 4049 bestimmt. Zur Bestimmung der Freisetzung von BVCPRE wurden analog hergestellte Prüfkörper (Durchmesser: 10 mm; Höhe: 2 mm) in 20 ml Ethanol bei 37 °C in einem Schüttler gelagert und nach 72 h der eluierbare Restmonomeranteil an BVCPRE mittels HPLC ermittelt.

**Tabelle 1**

| Zusammensetzung der Füllungskomposite | | |
|---|---|---|
| Bestandteil | Material A (Vergleichsbeispiel) (Gew.-%) | Material B (Gew.-%) |
| Urethandimethacrylat RM-3 | 7,47 | 7,47 |
| Bis-GMA | 8,38 | 8,38 |
| BVCPRE¹⁾ | 4,00 | 3,00 |
| Vinylcyclopropanmethacrylat (4) | - | 1,00 |
| Photoinitiator²⁾ | 0,18 | 0,18 |
| Ytterbiumtrifluorid³⁾ | 12,82 | 12,82 |
| Aerosil OX-50, sil.⁴⁾ | 1,03 | 1,03 |
| Ba-Glas, sil.⁵⁾ | 52,40 | 52,40 |
| Sphärosil, sil.⁶⁾ | 13,72 | 13,72 |

| | | |
|---|---|---|
| ¹⁾ Synthese vgl.: EP 0 798 286 A1 | | |
| ²⁾ Initiator: Campherchinon; Beschleuniger: N-(2-Cyanoethyl)-N-methylanilin; Inhibitor: Hydrochinonmonomethylether | | |
| ³⁾ Ytterbiumfluorid (Rhone-Poulenc) | | |
| ⁴⁾ Silanisierte Pyrolysckieselsäure (Degussa) | | |
| ⁵⁾ Silanisiertes Bariumaluminiumsilikatglaspulver (Schott) | | |
| ⁶⁾ Silanisiertes SiO₂-ZrO₂-Mischoxid (Tokoyama Soda) | | |

**Tabelle 2**

| Eigenschaften der Komposite | | |
|---|---|---|
| Materialeigenschaft | Material A (Vergleichsbeispiel) | Material B |
| Biegefestigkeit nach ISO 4049 (MPa) | 112 | 108 |
| Biege-E-Modul nach ISO 4049 (GPa) | 8800 | 10600 |
| Eluierbarer BVCPRE-Anteil (%) | 0,203 | 0,094 |

Aus Tabelle 2 ist ersichtlich, daß sich das Material B mit dem erfindungsgemäßen Vinylcyclopropanmethacrylat (4) durch eine signifikant bessere Vernetzung auszeichnet, was einerseits aus dem höheren E-Modul und andererseits aus dem deutlich geringeren eluierbaren BVCPRE-Restmonomeranteil abgeleitet werden kann.

## Patentansprüche

1. Vinylcylopropan-Derivat gemäß der Formel 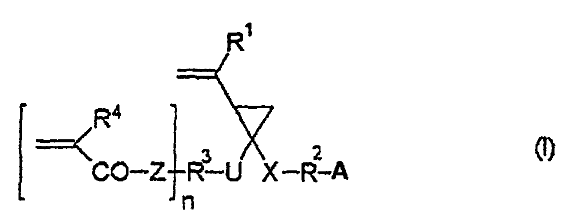 in der
A -[-Y-CO-C(=CH₂)R⁴]ₘ oder -C(=CH₂)CO-O-C₁₋₄-Alkyl ist;
U, X CO, COO oder CONH bedeuten;
Y, Z O oder NH bedeuten oder entfallen, wobei Y und Z nicht gleichzeitig entfallen können wenn A gleich -[-Y-CO-C(=CH₂)R⁴]ₘ ist;
n, m eine ganze Zahl von 0 bis 4 bedeuten, wobei n und m nicht gleichzeitig 0 sind;
R¹ H, CH₃ oder Cl ist;
R², R³ H, einen C₁- bis C₁₀-Alkyl- bzw. Alkylenrest, der durch O, S oder NH unterbrochen sein kann, einen C₆- bis C₁₄-Aryl- bzw. Arylen-, C₇- bis C₁₄-Alkylaryl-bzw. Alkylarylen oder einen C₅- bis C₈-Cycloalkyl-bzw. Cycloalkylenrest bedeuten; und
R⁴ H oder ein C₁- bis C₁₀-Alkylrest ist.

2. Vinylcyclopropan-Derivat gemäß Anspruch 1, **dadurch gekennzeichnet, daß**
A -[-Y-CO-C(=CH₂)R⁴]ₘ bedeutet,
U, X COO oder CONH bedeuten,
Y, Z O bedeuten oder entfallen,
n, m eine ganze Zahl von 0 bis 2 bedeuten,
R¹ H bedeutet,
R², R³ einen C₁- bis C₆-Alkyl- bzw. Alkylenrest, C₆-Aryl- bzw. Arylen- oder einen C₆-Cycloalkyl- bzw. Cycloalkylenrest bedeuten; und/oder
R⁴ H oder C₁- bis C₃-Alkylrest ist.

3. Vinylcyclopropan-Derivat gemäß Anspruch 2, **dadurch gekennzeichnet, daß**
R² und R³ gleich sind;
R⁴ H ist;
n,m jeweils 1 sind und
U, X COO oder CONH bedeuten,
oder
R² und R³ verschieden sind;
n 0 ist,
m 1 oder 2 ist,
Z entfällt,
Y O und
R⁴ H bedeutet.

4. Verfahren zur Herstellung von Vinylcyclopropan-Derivaten nach einem der Ansprüche 1 bis 3, **dadurch gekennzeichnet, daß** man ein Vinylcyclopropan-Derivat der Formel II bzw. VI, in der R⁵ ein C₁- bis C₁₀-Alkyl- oder Phenylrest ist, 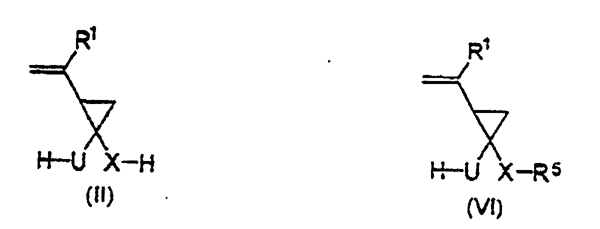 mit einem (Meth)acrylsäurederivat (III) in dem W die Bedeutung OH oder Halogen hat, 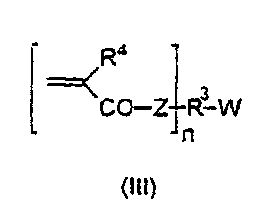 zu der Zwischenstufe (IV) bzw. der Endstufe (V) umsetzt, 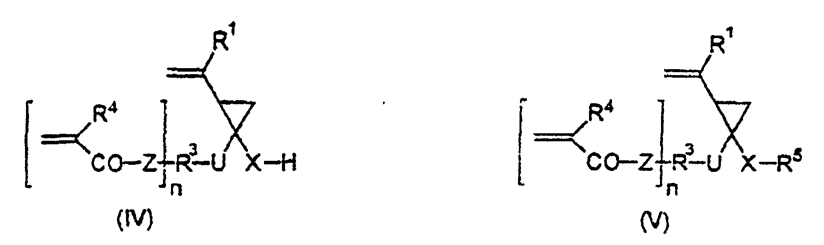 und ggf. (IV) mit einem weiteren (Meth)acrylsäurederivat (III), das mit dem ersten (Meth)acrylsäurederivat identisch sein kann, in das gewünschte Vinylcyclopropan-Derivat der Formel (Ia) überführt

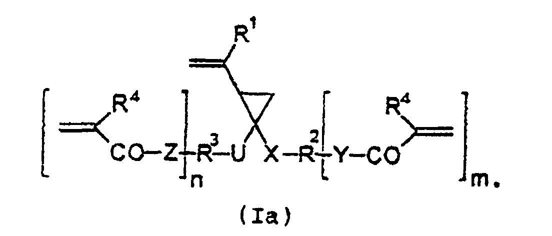

5. Dentalmaterial enthaltend
(a) 1 bis 99 Gew.-% eines Vinylcyclopropan-Derivats gemäß einem der Ansprüche 1 bis 3,
(b) 0 bis 80 Gew.-% eines weiteren radikalisch polymerisierbaren Polymers,
(c) 0,01 bis 5 Gew.-% eines Initiators für die radikalsiche Polymerisation
(d) 0 bis 90 Gew.-% Füllstoff.

6. Dentalmaterial gemäß Anspruch 5, **dadurch gekennzeichnet, daß** es
(a) 5 bis 70 Gew.-% eines Vinylcyclopropan-Derivats gemäß einem der Ansprüche 1 bis 3,
(b) 0 bis 60 Gew.-% eines weiteren radikalisch polymerisierbaren Polymers, und/oder
(c) 0,1 bis 2 Gew.-% eines Initiators für die radikalsiche Polymerisation enthält.

7. Dentalmaterial gemäß Anspruch 5 oder 6, **dadurch gekennzeichnet, daß** es 0 bis 20 Gew.-% (Adhäsiv), 20 bis 60 Gew.-% (Zement) oder 60 bis 85 Gew.-% (Füllungskomposit) Füllstoff enthält.

8. Verwendung eines Vinylcyclopropyl-Derivats gemäß einem der Ansprüche 1 bis 3 zur Herstellung von Adhäsiven, Zementen, Kompositen oder Formkörpern.

9. Verwendung gemäß Anspruch 8 zur Herstellung von Dentalmaterialien.

## Claims

1. Vinylcyclopropane derivative according to the formula: 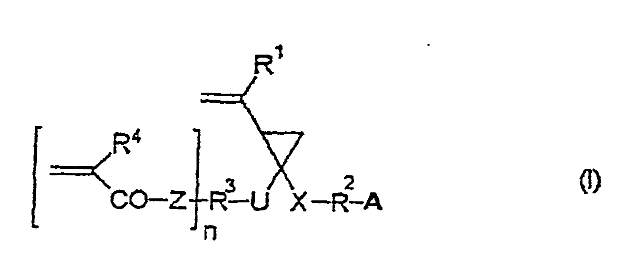 in which
A is -[-Y-CO-C(=CH₂)R⁴]ₘ or -C(=CH₂)CO-O-C₁₋₄-alkyl;
U, X stand for CO, COO or CONH;
Y, Z stand for O or NH or are absent, but Y and Z cannot be absent at the same time when A is -[Y-CO-C(=CH₂)R⁴]ₘ;
n, m stand for an integer from 0 to 4, but n and m are not 0 at the same time;
R¹ is H, CH₃ or Cl;
R², R³ stand for H, a C₁- to C₁₀-alkyl or alkylene radical, which can be interrupted by O, S or NH, for a C₆- to C₁₄-aryl or arylene, C₇- to C₁₄-alkylaryl or alkylarylene or a C₅- to C₈-cycloalkyl or cycloalkylene radical; and
R⁴ is H or a C₁- to C₁₀-alkyl radical.

2. Vinylcyclopropane derivative according to Claim 1, **characterized in that**
A stands for -[-Y-CO-C(=CH₂)R⁴]ₘ,
U, X stand for COO or CONH,
Y, Z stand for O or are absent,
n, m stand for an integer from 0 to 2,
R¹ stands for H,
R², R³ stand for a C₁- to C₆-alkyl or alkylene radical, C₆-aryl or arylene or a C₆-cycloalkyl or cycloalkylene radical; and/or
R⁴ is H or a C₁- to C₃-alkyl radical.

3. Vinylcyclopropane derivative according to Claim 2, **characterized in that**
R² and R³ are the same;
R⁴ is H;
n, m are in each case 1 and
U, X stand for COO or CONH,
or
R² and R³ are different;
n is 0,
m is 1 or 2,
Z is absent,
Y stands for O and
R⁴ stands for H.

4. Process for the production of vinylcyclopropane derivatives according to one of Claims 1 to 3, **characterized in that** a vinylcyclopropane derivative of the formula II or VI, in which R⁵ is a C₁- to C₁₀-alkyl or phenyl radical, 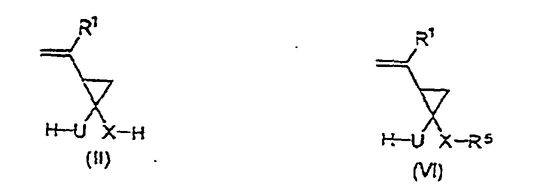 is reacted with a (meth)acrylic acid derivative (III) in which W has the meaning OH or halogen, 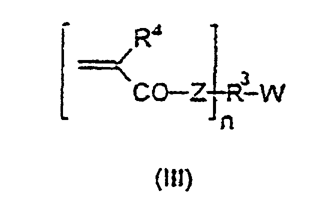 to give the intermediate stage (IV) or the final stage (V), 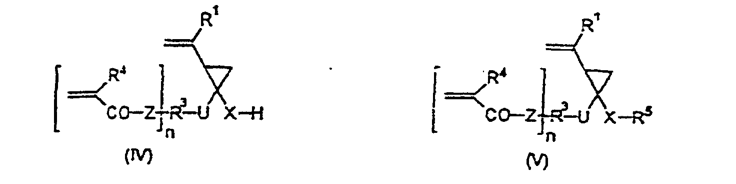 and (IV) is optionally converted with a further (meth)acrylic acid derivative (III), which can be identical with the first (meth)acrylic acid derivative, into the desired vinylcyclopropane derivative of the formula (Ia)

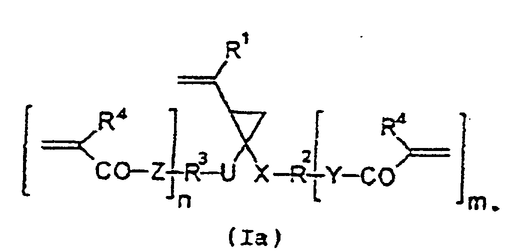

5. Dental material containing
(a) 1 to 99 wt.-% of a vinylcyclopropane derivative according to one of Claims 1 to 3,
(b) 0 to 80 wt.-% of another radically polymerizable polymer,
(c) 0.01 to 5 wt.-% of an initiator for the radical polymerization,
(d) 0 to 90 wt.-% of a filler.

6. Dental material according to Claim 5, **characterized in that** it contains
(a) 5 to 70 wt.-% of a vinylcyclopropane derivative according to one of Claims 1 to 3,
(b) 0 to 60 wt.-% of another radically polymerizable polymer, and/or
(c) 0.1 to 2 wt.-% of an initiator for the radical polymerization.

7. Dental material according to Claim 5 or 6, **characterized in that** it contains 0 to 20 wt.-% of an adhesive, 20 to 60 wt.-% of a cement or 60 to 85 wt.-% of a filling composite filler.

8. Use of a vinylcyclopropyl derivative according to one of Claims 1 to 3 for the production of adhesives, cements, composites or mouldings.

9. Use according to Claim 8 for the production of dental materials.

## Revendications

1. Dérivé de vinylcyclopropane de formule : 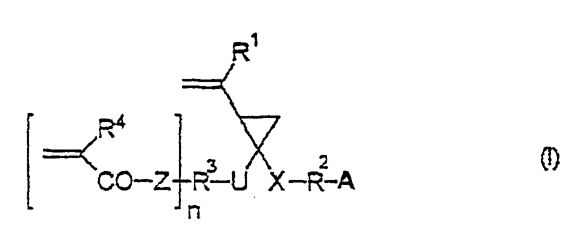 dans laquelle :
A désigne -[-Y-CO-C(=CH₂)R⁴]ₘ ou -C(=CH₂)CO-O-alkyle en C₁₋₄;
U, X désignent CO, COO ou CONH;
Y, Z désignent O ou NH ou sont absents, Y et Z ne pouvant simultanément être absents lorsque A est égal à -[-Y-CO-C(=CH₂)R⁴]ₘ;
n, m désignent un nombre entier de 0 à 4, n et m n'étant pas simultanément égaux à 0;
R¹ désigne H, CH₃ ou Cl;
R², R³ désignent H, un groupe alkyle ou alkylène en C₁-C₁₀, qui peut être interrompu par O, S ou NH, aryle ou arylène en C₆-C₁₄, alkylaryle ou alkylarylène en C₇-C₁₄ ou cycloalkyle ou cycloalkylène en C₅-C₈; et
R⁴ est H ou un groupe alkyle en C₁-C₁₀.

2. Dérivé de vinylcyclopropane selon la revendication 1, **caractérisé en ce que** :
A désigne -[-Y-CO-C(=CH₂)R⁴]ₘ,
U, X désignent COO ou CONH,
Y, Z désignent O ou sont absents,
n, m désignent un nombre entier de 0 à 2,
R¹ désigne H,
R², R³ désignent un groupe alkyle ou alkylène en C₁-C₆, aryle ou arylène en C₆ ou cycloalkyle ou cycloalkylène en C₆; et/ou
R⁴ est H ou un groupe alkyle en C₁-C₃.

3. Dérivé de vinylcyclopropane selon la revendication 2, **caractérisé en ce que** :
R² et R³ sont identiques;
R⁴ est H;
n, m désignent respectivement 1, et
U, X désignent COO ou CONH,
ou
R² et R³ sont différents,
n est 0,
m est 1 ou 2,
Z est absent,
Y est O, et
R⁴ désigne H.

4. Procédé de fabrication de dérivés de vinycyclopropane selon l'une quelconque des revendications 1 à 3, **caractérisé en ce que** l'on convertit un dérivé de vinylcyclopropane de formule II ou VI, dans lequel R⁵ est un groupe alkyle en C₁-C₁₀ ou phényle : 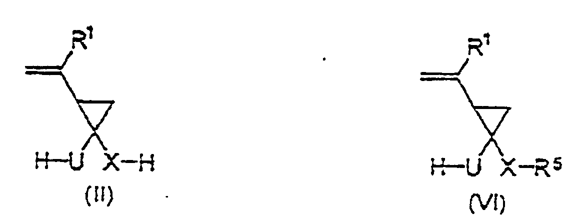 avec un dérivé d'acide (méth)acrylique (III), dans lequel W désigne OH ou un halogène 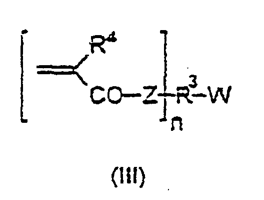 en produit intermédiaire (IV) ou en produit final (V) : 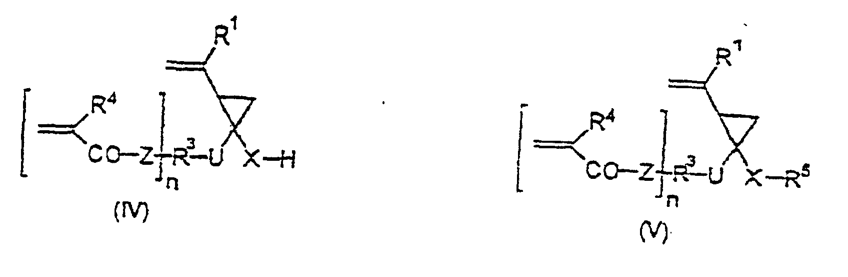 et éventuellement on transforme (IV) avec un autre dérivé d'acide (méth)acrylique (III), qui peut être identique au premier dérivé d'acide (méth)acrylique, en dérivé de vinylcyclopropane souhaité de formule (Ia) :

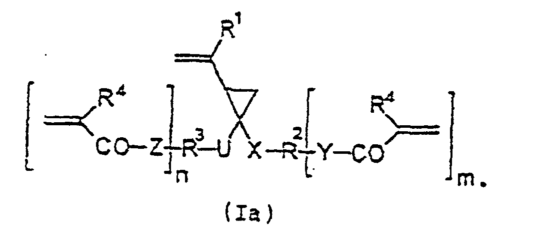

5. Matériau dentaire contenant :
(a) 1% à 99% en poids d'un dérivé de vinylcyclopropane selon l'une quelconque des revendications 1 à 3,
(b) 0 à 80% en poids d'un autre polymère à polymérisation radicalaire,
(c) 0,01% à 5% en poids d'un initiateur pour la polymérisation radicalaire, et
(d) 0 à 90% en poids de charge.

6. Matériau dentaire selon la revendication 5, **caractérisé en ce qu'**il contient :
(a) 5% à 70% en poids d'un dérivé de vinylcyclopropane selon l'une quelconque des revendications 1 à 3,
(b) 0 à 60% en poids d'un autre polymère à polymérisation radicalaire, et/ou
(c) 0,1% à 2% en poids d'un initiateur pour la polymérisation radicalaire,

7. Matériau dentaire selon la revendication 5 ou 6, **caractérisé en ce qu'**il contient 0 à 20% en poids (adhésif), 20% à 60% en poids (ciment) ou 60% à 85% en poids (composite de remplissage) de charge.

8. Utilisation d'un dérivé de vinylcyclopropyle selon l'une quelconque des revendications 1 à pour la fabrication d'adhésifs, de ciments, de composites ou de corps moulés.

9. Utilisation selon la revendication 8 pour la fabrication de matériaux dentaires.
